# EUROPÄISCHE PATENTSCHRIFT

(11) **EP 1 040 180 B1**
(45) Veröffentlichungstag und Bekanntmachung des Hinweises auf die Patenterteilung: **06.05.2004**
(21) Anmeldenummer: 98966491.7
(22) Anmeldetag: 19.11.1998
(51) Int. Cl.: C12M 1/04

(54) **VERFAHREN UND VORRICHTUNG ZUR BEGASUNG VON MEDIEN SOWIE BEGASUNGSKÖRPER**
METHOD AND DEVICE FOR GASSING AND GASIFICATION BODY
PROCEDE ET DISPOSITIF POUR LA FUMIGATION DE MILIEUX

(30) Priorität: 28.11.1997 DE 19752604
(43) Veröffentlichungstag der Anmeldung: 04.10.2000
(73) Patentinhaber: Forschungszentrum Jülich GmbH, 52425 Jülich (DE); Infors AG, 4103 Bottmingen (CH)
(72) Erfinder: ALTENBACH-REHM, Jutta, D-67065 Ludwigshafen (DE); WEUSTER-BOTZ, Dirk, D-85221 Dachau (DE); HAWRYLENKO, Alexander, CH-4103 Bottmingen (CH)
(86) Internationale Anmeldenummer: PCT/DE1998/003447
(87) Internationale Veröffentlichungsnummer: WO 1999/028435

(56) Entgegenhaltungen:
- FR-A- 709 948
- FR-A- 710 667
- FR-A- 787 817
- FR-A- 851 558
- FR-A- 1 348 795
- FR-A- 2 406 663
- GB-A- 340 644
- GB-A- 701 342
- GB-A- 712 170

## Beschreibung

Die Erfindung betrifft ein Verfahren zur Begasung von Medien, eine für die Durchführung der Begasung geeignete Vorrichtung, sowie einen Begasungskörper.

Für die Kultivierung von Mikroorganismen ist es notwendig, Gas in Reaktoren oder Gefäße, welche die Mikroorganismen beinhalten, einzutragen. Zu diesem Zweck ist aus der DE-OS 195 29 099 eine Vorrichtung zur Serienkultivierung in begasten Flüssigkeitssäulen bekannt, bei der Gas durch eine poröse Sinterglasbodenplatte, welche mit hydrophobem Material beschichtet ist, in eine Kulturflasche eingebracht wird.

Mit der Vorrichtung der DE-OS 195 29 099 werden zwar recht gute Begasungsergebnisse erzielt, jedoch ist es wünschenswert, die Effektivität der Begasung zu vergrößern, um den Gasverbrauch zu senken.

Das französische Patent 710.667 offenbart einen Begasungskörper mit Öffnungen eines Durchmessers von 0,2 bis 0,4 mm, die sich gleichmäßig über die Oberfläche des Begasungskörpers verteilen.

Das französische Patent 851.558 offenbart einen Begasungskörper mit sich konisch verjüngenden Gasaustrittsöffnungen.

Es ist daher Aufgabe der Erfindung, ein Verfahren und eine Vorrichtung zu schaffen, mit denen die Begasungseffektivität vergrößert werden kann.

Ausgehend vom Oberbegriff des Anspruchs 1 wird die Aufgabe erfindungsgemäß gelöst, mit dem im kennzeichnenden Teil des Anspruchs 1 angegebenen Merkmalen.

Mit dem erfindungsgemäßen Verfahren und der Vorrichtung ist es nunmehr möglich eine Begasung mit einem höheren Wirkungsgrad durchzuführen.

Vorteilhafte Weiterbildungen der Erfindung sind in den Unteransprüchen angegeben.

Die Zeichnung zeigt eine erfindungsgemäße Vorrichtung in schematischer Form.

Es Zeigt:

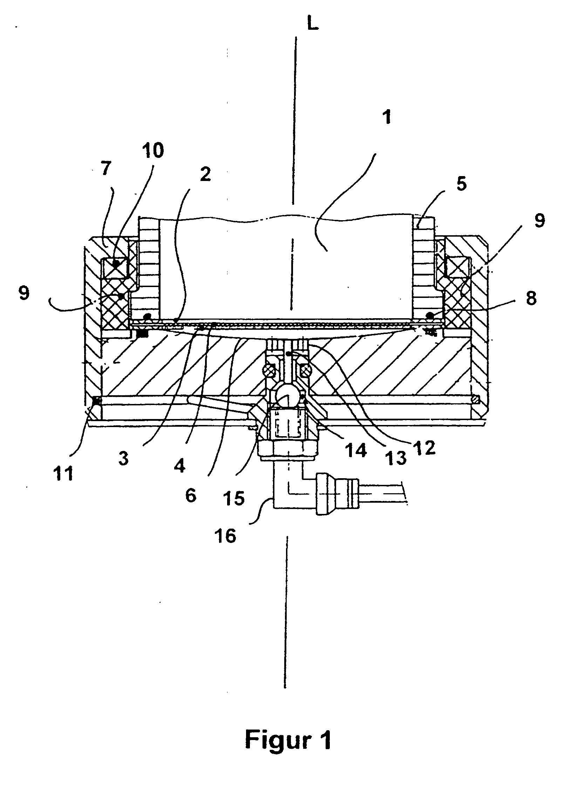

- Fig.1:: Eine Vorrichtung zur Begasung mit einer Begasungsplatte.

Figur 1 zeigt eine erfindungsgemäße Vorrichtung, die mit einem Gefäß 1 und mit einem als Begasungsplatte 2 ausgebildetem Begasungkörper ausgestattet ist. Unter der Begasungsplatte 2 befindet sich ein Flachmembranfilter 3. Zwischen dem Flachmembranfilter 3 und der Begasungsplatte 2 ist ein Stützgewebe 4 aufgenommen. Die Begasungsplatte 2, das Stützgewebe 4 sowie das Flachmembranfilter 3 werden zwischen den Wänden 5 des Gefäßes 1 und dessen Boden 6 durch eine Flanschverbindung 7 pressschlüssig aufgenommen. Die Flanschverbindung 7 nimmt zusätzlich einen O-Ring 8 auf. Zwischen der Flanschverbindung 7 und einem Stützring 9 befindet sich eine Sinuns-Flachfeder 10, die durch einen Sperring 11 arretiert wird. An dem leicht nach unten gewölbten Boden 6 befindet sich ein Stutzen 12, welcher einen Stift 13 aufweist, der in ein Ventil 14 mit einer Verschlußkugel 15 eingreift. Das Ventil 14 führt sich in einer Leitung 16 fort, welche eine Gaszufuhr ermöglicht.

Bei Betrieb wird das Gefäß 1 mit der zu begasenden Lösung beschickt und mit dem Stutzen 12 auf das Ventil 14 aufgesetzt. Dabei tritt der Stift 13 in das Ventil 14 ein und drückt die Verschlußkugel 15 herunter. Dabei wird ein Gasstrom freigegeben, der durch die Leitung 16 eingeführt wird, und die Verschlußkugel 15 umströmt. Der Gasstrom gelangt entlang der Wegstrecke um den Stift 13 in den Stutzen 12 und anschließend in den Bodenbereich des Gefäßes 1. Der Gasstrom durchtritt anschließend das Flachmembranfilter 3, das Stützgewebe 4 sowie die Begasungsplatte 2. Das Gas gelangt somit in den mit Flüssigkeit gefüllten Innenraum des Gefäßes 1. Erfindungsgemäß tritt das Gas durch den als Begasungsplatte 2 ausgebildeten Begasungskörper in die Flüssigkeit ein, der Poren mit gleicher oder im wesentlichen gleicher Größe aufweist, die hochpräzise gebohrt sind und eine geringe Toleranz von < 10% im Durchmesser haben. Der Durchmesser der Poren kann Werte zwischen 10 µm und 120 µm annehmen. Dies hat zur Folge, daß keine Inhomogenität der Blasengröße beim Durchtritt durch die Begasungsplatte 2 auftritt, wie sie bei Begasungsverfahren nach dem Stand der Technik beobachtet wird. Eine Begasung ist somit besonders effektiv und mit gleichmäßiger Verteilung möglich, wodurch letztendlich eine Einsparung von Gas erfolgt. Unter einer hochpräzisen Bohrung einer Pore im Sinne der Erfindung ist idealerweise eine kreisförmige Bohrung einer Durchtrittsfläche, die einem Kreisdurchmesser von 10-120 µm entspricht zu versthen. Aus fertigunstechnischen Gründen kann die Form der Pore jedoch von der idealen Kreisform abweichen, so daß beispielsweise eine elypsoide Geometrie der Pore auftritt. In diesem Fall soll die Durchtrittsfläche ebenfalls so groß sein, daß sie auf die Kreisgeometrie umgerechnet einer Durchtrittsfläche des Kreises eines Durchmessers von 10-120 µm entspricht. Der Fehler in der Durchtrittsfläche soll jedenfalls eine geringe Toleranz aufweisen und gemessen an der Kreisform < 10% des Durchmessers, beispielsweise 1,5 µm sein. Diese Poren sollen von der Erfindung mit umfaßt sein. Durch die Dimensionierung der Gasbläschen ist das Verhältnis zwischen Volumen und Oberfläche groß, so daß in Bezug auf die eingetragene Gasmenge eine schnelle Löslichkeit erfolgt. Somit wird eine maximale Anzahl möglichst kleiner Gasblasen in einem weiten Gasströmungsbereich erhalten. Mit steigendem Gasdruck kann die Porengröße auch kleiner als 10 µm , zum Beispiel 1 µm sein, jedoch werden an die Genauigkeit der Porengröße höhere Anforderungen gestellt. Bei der Herstellung der Poren mit einem Laser werden aus fertigungstechnischen Gründen Porengrößen von 20-100 µm bevorzugt. Der Durchmesser der Gasblasen entspricht etwa den 10-fachen Porendurchmesser. In einer bevorzugten Ausführungsform haben die Poren einen minimalen Abstand des 10-fachen Durchmessers der Poren. Hierdurch wird eine Koaleszenz der Gasbläschen verhindert, was die Gleichmäßigkeit und Effektivität der Begasung zusätzlich unterstützt. Dieser Wert ist jedoch von der Wahl des Flüssigen Mediums abhängig und bezieht sich in diesem Fall auf Wasser, welches als Lösungsmittel im Bereich der Kultivierung von Mikroorganismen eingesetzt wird. Selbstverständlich können für andere Begasungsprobleme, wie beim Eintrag von Gas in eine Lösung von Chemikalien zur Durchführung einer chemischen Reaktion, auch andere Lösungsmittel zum Einsatz kommen, welche eine andere Viskosität und Oberflächenspannung aufweisen. In diesen Fällen wird ein anderer minimaler Abstand notwendig sein, der mit abnehmender Viskosität und Oberflächenspannung des Lösungsmittel zunimmt.
Die Begasungsplatte 2 kann bevorzugt aus hydrophilen Materialien ausgebildet sein, wenn das Lösungsmittel polar ist. Dies hat zur Folge daß sich die Gasbläschen besonders gut von der Austrittsoberfläche abtrennen und dadurch besonders klein ausfallen. Nach dem Stand der Technik war dies nicht möglich, da die Oberflächen zur Vermeidung des Flüssigkeitsaustrittes nach unten hydrophob ausgebildet oder beschichtet waren. Erfindungsgemäß ist mindestens die Austrittsoberfläche des Begasungskörpers oder der gesamte Begasungskörper aus einem Material mit gleicher oder ähnlicher Polarität, wie das Lösungsmittel, so das bei der Verwendung von Wasser eine hydrophile Oberfläche des Begasungskörpers und bei Verwendung von unpolaren Lösungsmitteln eine hydrophobe Oberfläche des Begasungskörpers zum Einsatz kommt. Vorzugsweise besteht die Begasungsplatte 2 beim Betrieb der Vorrichtung mit polarem Lösungsmittel aus metallischen Werkstoffen, wie Stahl, es sind jedoch auch keramische Materialien, wie Glas möglich.

In einer weiteren bevorzugten Ausführungsform sind die Poren zur Austrittsoberfläche hin konisch zulaufend ausgebildet. Im Vergleich zum Stand der Technik hat sich überraschenderweise gezeigt, daß mit zur Austrittsoberfläche hin konisch zulaufend ausgebildeten Poren versehene Begasungskörper bei der kultivierung von Pilzen keine Mycelbildung im Begasungskörper zeigen, was zu einem besseren Durchsatz führt, da die Poren nicht verstopfen.

Das Flachmembranfilter 3 dient zur Sterilisierung des in den Innenraum des Gefäßes 1 eintretenden Gases. Der Porendurchmesser eines Filters ist vorzugsweise < 0,45 µm. Für den Betrieb der Vorrichtung mit wäßrigen Medium besteht das Flachmembranfilter 3 aus hydrophobem Material oder ist mindestens an seiner Oberfläche mit hydrophobem Material beschichtet. Dies ermöglicht, daß das wäßrige Medium ohne Gaszufuhr nicht nach unten austritt und aus dem Gefäß 1 ausläuft. Auf eine hydrophobe Ausbildung der Oberfläche der Begasungsplatte 2 kann somit verzichtet werden, was zu oben genanntem Vorteil der hydrophilen Eigenschaften der Begasungsplatte 2 führt. Das Flachmembranfilter 3 hat somit in Sinne der Erfindung die umgekehrten Eigenschaften bezüglich der Hydrophilie beziehungsweise der Hydrophobie wie die Begasungsplatte 2 und somit auch des Lösungsmittels, was zur Folge hat, daß das Lösungsmittel nicht durch das Filter nach unten durchtritt.

Zwischen dem Flachmembranfilter 3 und der Begasungsplatte 2 befindet sich das Stützgewebe 4. Der Einsatz des Stützgewebes 4 führt dazu, daß die Gastransportkapazität des Flachmembranfilters 3 auch an den Stellen genutzt wird, die nicht direkt unterhalb einer Pore der Begasungsplatte 2 liegen.

Für sterile Zwecke müssen die Begasungsplatte 2, das Stützgewebe 4 sowie das Flachmembranfilter 3 absolut dicht mit den Wänden 5 des Gefäßes 1 abschließen. Die Vorrichtung ist autoklavierbar. In einer bevorzugten Ausführungsform, bei der die Einzelteile ausgewechselt werden können, ist dies durch ein Fixieren dieser Teile in einer Flanschverbindung 7 gewährleistet, die vorzugsweise einen O-Ring 8 einschließt. Für sterile Zwekke ist die Vorspannung der Sinus-Flachfeder 10 so zu bemessen, daß sowohl die während der Sterilisation auftretenden Diletationseinflüsse, als auch die notwendigen Dichtungskräfte stets innerhalb der zulässigen bzw. erforderlichen Werte bleiben. Diese Konstruktion in Verbindung mit dem O-Ring 8 erlaubt einen leichten und mehrmaligen Austausch der Begasungsplatte 2 sowie des Stützgewebes 4 und des empfindlichen Flachmembranfilters 3 ohne letzteres zu beschädigen. Generell ist jede verschraubungsfreie Befestigung für die Begasungsplatte 2, das Flachmembranfilter 3 und das Stützgewebe 4 geeignet, da es hierbei nicht zu Verwerfungen im Flachmembranfiltermaterial kommt, die zu mit dem Gefäßrand unschlüssigen Lücken oder sogar zur Zerstörung des Filters führen können. Für nicht-sterile Anwendungen ist eine verschraubungsfreie Halterung für die Begasungsplatte 2, das Stützgewebe 4 und die Flachmembranfilter 3 nicht zwingend nötig. In diesem Fall können auch andere Filterelemente höherer Festigkeit eingesetzt werden. Diese sollen wiederum umgekehrte Eigenschaften bezüglich der Hydrophilie beziehungsweise der Hydrophobie aufweisen, wie die Begasungsplatte 2 und unter den Begriff Flachmembranfilter subsummiert werden.

In einer weniger bevorzugten Ausführungsform können die Begasungsplatte 2, das Flachmembranfilter 3 sowie das Stützgewebe 4 auch nicht-austauschbar an den Wänden 5 des Gefäßes 1 angebracht sein. In diesem Fall können diese Teile zum Beispiel in die Glaswände des Gefäßes 1 eingegossen sein, sofern das Gefäß 1 aus Glas besteht. In einer weiteren bevorzugten Ausführungsform ist der Boden 6 derart ausgebildet, daß er keine Abrißkanten für das Flachmembranfilter 3 bildet. Dies ist insbesondere dann von Vorteil, wenn die erfindungsgemäße Vorrichtung über die Leitung 16 mit Heißdampf sterilisiert wird und dabei Druckschwankungen unterliegt. Ist der Druck im Innenraum des Gefäßes 1 höher als außerhalb, so kommt es zu einer Ausdehnung des Flachmembranfilters 3 zur Bodenseite der Vorrichtung hin. Daher wölbt sich der Boden 6 zur Mitte hin nach unten und schließt an der Wänden 5 des Gefäßes 1 ohne Höhendifferenz zum Flachmembranfilter 3 ab. Bei Beaufschlagung des Gefäßinnenraumes mit Druck legt sich das Flachmembranfilter 3 somit an den Boden 6 zerstörungsfrei an und wird somit entlastet. Jedenfalls darf das Material des Flachmembranfilters 3 keinen sprunghaften Höhendifferenzen entlang der Längsachse L der Vorrichtung ausgesetzt sein, die einen Abriß bewirken.

Wird das Gefäß 1 vom als Steckventil ausgebildetem Ventil 14 abgezogen, so schließt dieses automatisch. Damit wird jeder Gasverlust vermieden. Durch das Anbringen von mehreren Ventilen 14 an eine gemeinsame Leitung 16 können auf bequeme Weise mehrere Gefäße 1 parallel betrieben werden.

Der erfindungsgamäße Begasungskörper ist vorzugsweise als Begasungsplatte 2 ausgebildet und am Boden 6 einer Vorrichtung zur Begasung angebracht. Jedoch kann er in anderen Ausführungsformen eine andere Geometrie und Positionierung aufweisen. So können auch die Wände 5 eines Reaktors mit dem Begasungskörper ausgekleidet sein oder ein am Boden 6 angebrachter Begasungskörper kann eine Wölbung ausfweisen, so daß die Oberfläche vergrößert ist.

Mit der erfindungsgemäßen Vorrichtung und dem Begasungskörper ist ein besserer Stofftransport bei der sterilen Begasung von Flüssigkeiten durch optimale Bohrungsgeometrie und Bohrungsverteilung möglich.

Für den Begasungskörper kann hydrophiles Material eingesetzt werden, welches beim Betrieb von Begasungsreaktoren für in wässrigem Medium suspendierte Mikroorganismen zu einer besonders guten Begasung führt, da die Abdichtung des Reaktors nach unten hin durch die hydrophobe Membran bewirkt wird.

Die Verstopfungsgefahr des Begasungskörpers z.B. durch mycelbildende Organismen ist stark vermindert bzw. es wird keine Mycelbildung in den Poren mehr beobachtet. Die Handhabung der Vorrichtung ist durch die Steckventile besonders einfach und gassparend und es ist ein Parallelbetrieb von mehreren Reaktoren möglich.

Durch die gerade, axiale Anpressung der Flanschverbindung sowie die Ausbildung des Bodens 6 besteht keine Kontaminationsgefahr durch Keime, da das emfpindliche Flachmembranfilter 3 nicht beschädigt wird.

Die erfindungsgemäße Vorrichtung kann im biotechnologischen und chemischen Bereich, sowie in der Umweltforschung eingesetzt werden.

## Patentansprüche

1. Begasungskörper (2) mit Poren,
**dadurch gekennzeichnet,**
- **dass** die Poren eine im wesentlichen gleiche Größe haben und
- der Durchmesser der Poren einen Wert zwischen 10 µm und 120 µm aufweist mit einer geringen Toleranz von < 10 % aufweist und
- **dass** der Abstand der Poren mindestens dem 10-fachen Porendurchmesser entspricht und
- **dass** die Poren denselben Abstand zueinander haben.

2. Begasungskörper (2) nach Anspruch 1,
**dadurch gekennzeichnet,**
**dass** er mindestens an seiner Austrittsoberfläche aus hydrophilem Material ausgebildet ist.

3. Begasungskörper (2) nach einem der Ansprüche 1 oder 2,
**dadurch gekennzeichnet,**
**dass** die Poren in Richtung der Austrittsoberfläche konisch zulaufen.

4. Begasungskörper (2) nach einem der Ansprüche 1 bis 3,
**dadurch gekennzeichnet,**
**dass** er als Begasungsplatte (2) ausgebildet ist.

5. Vorrichtung zur sterilen Begasung von Medien, umfassend ein Gefäß (1) mit einem Begasungskörper (2),
**dadurch gekennzeichnet,**
**dass** der Begasungskörper (2) nach den Merkmalen eines der Ansprüche 1 bis 4 ausgebildet ist.

6. Vorrichtung nach Anspruch 5,
**dadurch gekennzeichnet,**
**dass** unterhalb des Begasungskörpers (2) ein Flachmembranfilter (3) angebracht ist.

7. Vorrichtung nach Anspruch 6,
**dadurch gekennzeichnet,**
**dass** das Flachmembranfilter (3) bezüglich der Hydrophilie und Hydrophobie umgekehrte Eigenschaften wie der Begasungskörper (2) hat.

8. Vorrichtung nach Anspruch 6 oder 7,
**dadurch gekennzeichnet,**
**dass** das Flachmembranfilter (3) hydrophob ist.

9. Vorrichtung nach einem der Ansprüche 6 bis 8,
**dadurch gekennzeichnet,**
**dass** das Flachmembranfilter (3) eine Porengröße von < 0,45 µm aufweist.

10. Vorrichtung nach einem der Ansprüche 6 oder 9,
**dadurch gekennzeichnet,**
**dass** sich zwischen dem Begasungskörper (2) und dem Flachmembranfilter (3) ein Stützgewebe (4) befindet.

11. Vorrichtung nach einem der Ansprüche 5 bis 10,
**dadurch gekennzeichnet,**
**dass** sie einen Boden (6) aufweist, welcher derart ausgebildet ist, daß sich das Flachmembranfilter (3) bei Druckschwankungen zerstörungsfrei ausdehnen kann.

12. Vorrichtung nach Anspruch 11,
**dadurch gekennzeichnet,**
**dass** der Boden (6) leicht nach unten gewölbt ist.

13. Vorrichtung nach einem der Ansprüche 5 bis 12,
**dadurch gekennzeichnet,**
**dass** sich an den Boden ein Ventil (14) anschließt, welches bei Aufsetzen des Gefäßes (1) einen Gasdurchtritt freigibt.

14. Vorrichtung nach Anspruch 13,
**dadurch gekennzeichnet,**
**dass** das Ventil (14) ein Steckventil mit einem Stift (13) ist, welcher auf eine Verschlußkugel (15) Druck ausübt, wodurch eine Gasstrom freigegeben wird.

15. Vorrichtung nach einem der Ansprüche 5 bis 14,
**dadurch gekennzeichnet,**
**dass** sie Mittel umfaßt, die den Begasungskörper (2) pressschlüssig mit dem Boden (6) des Gefäßes (1) in Kontakt bringen.

16. Verfahren zum Eintrag von Gas in ein Medium,
**dadurch gekennzeichnet,**
**dass** das Gas durch einen Begasungskörper (2) nach einem der Ansprüche 1 bis 4 eingetragen wird.

## Claims

1. Gassing body (2) having pores, **characterised in that**
- the pores are of substantially uniform size and
- the diameter of the pores is of a value between 10 µm and 120 µm with a slight tolerance of < 10% and
- the distance between the pores corresponds to at least 10 times the pore diameter and
- the pores are equally spaced from each other.

2. Gassing body (2) according to claim 1, **characterised in that** it is formed at least at its outlet surface from hydrophilic material.

3. Gassing body (2) according to one of claims 1 and 2, **characterised in that** the pores converge conically in the direction of the outlet surface.

4. Gassing body (2) according to one of claims 1 to 3, **characterised in that** it is formed as a gassing plate (2).

5. Device for the sterile gassing of media which includes a vessel (1) having a gassing body (2), **characterised in that** the gassing body (2) conforms to the features of one of claims 1 to 4.

6. Device according to claim 5, **characterised in that** a flat membrane filter (3) is arranged below the gassing body (2).

7. Device according to claim 6, **characterised in that** the flat membrane filter (3), as regards hydrophily and hydrophobia, has properties which are the opposite to those of the gassing body (2).

8. Device according to claim 6 or 7, **characterised in that** the flat membrane filter (3) is hydrophobic.

9. Device according to one of claims 6 to 8, **characterised in that** the flat membrane filter (3) has a pore size of < 0.45 µm.

10. Device according to one of claims 6 or 9, **characterised in that** a support fabric (4) is disposed between the gassing body (2) and the flat membrane filter (3).

11. Device according to one of claims 5 to 10, **characterised in that** it has a base (6) which is formed such that, upon variations in pressure, the flat membrane filter (3) can expand non-destructively.

12. Device according to claim 11, **characterised in that** the base (6) is slightly downwardly curved.

13. Device according to one of claims 5 to 12, **characterised in that**, in communication with the base, there is a valve (14) which when placed upon the vessel (1) permits the passage of gas.

14. Device according to claim 13, **characterised in that** the valve (14) is a push valve with a pin (13) that exerts pressure on a closure ball (15) thereby permitting the gas to flow.

15. Device according to one of claims 5 to 14, **characterised in that** it includes means for bringing the gassing body (2) into pressure closure contact with the base (6) of the vessel (1).

16. Method for introducing gas into a medium, **characterised in that** the gas is introduced by way of a gassing body (2) according to one of claims 1 to 4.

## Revendications

1. Pièce (2) de fumigation ayant des pores,
**caractérisée**
- **en ce que** les pores ont sensiblement la même dimension, et
- le diamètre des pores a une valeur comprise entre 10 µm et 120 µm avec une petite tolérance inférieure à 10 % , et
- la distance entre les pores représente au moins 10 fois le diamètre des pores, et
- les pores sont à la même distance les uns des autres.

2. Pièce (2) de fumigation suivant la revendication 1,
**caractérisée**
**en ce qu'**il est en un matériau hydrophile au moins sur sa surface de sortie.

3. Pièce (2) de fumigation suivant l'une des revendications 1 ou 2,
**caractérisée**
**en ce que** les pores se rétrécissent coniquement en direction de la surface de sortie.

4. Pièce (2) de fumigation suivant l'une des revendications 1 à 3,
**caractérisée**
**en ce qu'**elle est constituée en plaque (2) de fumigation.

5. Dispositif de fumigation stérile de milieux, comprenant un récipient (1) ayant une pièce (2) de fumigation,
**caractérisé**
**en ce que** la pièce (2) de fumigation est constituée suivant les caractéristiques de l'une des revendications 1 à 4.

6. Dispositif suivant la revendication 5,
**caractérisé**
**en ce qu'**un filtre (3) à membrane plat est disposé en dessous de la pièce (2) de fumigation.

7. Dispositif suivant la revendication 6,
**caractérisé**
**en ce que** le filtre (3) à membrane plat a pour ce qui concerne l'hydrophilie et l'hydrophobie des propriétés inverses de la pièce (2) de fumigation.

8. Dispositif suivant la revendication 6 ou 7,
**caractérisé**
**en ce que** le filtre (3) à membrane plat est hydrophobe.

9. Dispositif suivant l'une des revendications 6 à 9,
**caractérisé**
**en ce que** le filtre (3) à membrane plat a une dimension de pores inférieure à 0,45 µm.

10. Dispositif suivant l'une des revendications 6 ou 9,
**caractérisé**
**en ce qu'**il y a entre la pièce (2) de fumigation et le filtre (3) à membrane plat, un tissu (4) d'appui.

11. Dispositif suivant l'une des revendications 5 à 10,
**caractérisé**
**en ce qu'**il a un fond (6) qui est constitué de manière à ce que le filtre (3) à membrane plat puisse s'allonger sans destruction en cas de fluctuation de pression.

12. Dispositif suivant la revendication 11,
**caractérisé**
**en ce que** le fond (6) et légèrement bombé vers le bas.

13. Dispositif suivant l'une des revendications 5 à 12,
**caractérisé**
**en ce qu'**au fond se raccorde une vanne (14) qui, lorsque le récipient (1) est dressé, autorise un passage du gaz.

14. Dispositif suivant la revendication 13,
**caractérisé**
**en ce que** la vanne (14) est une vanne à pointeau (13), qui applique une pression sur une bille (15) obturatrice, grâce à quoi un courant de gaz peut passer.

15. Dispositif suivant l'une des revendications 5 à 14,
**caractérisé**
**en ce qu'**il comprend des moyens qui mettent la pièce (2) de fumigation avec pressage en contact avec le fond (6) du récipient (1 ).

16. Procédé d'introduction de gaz dans un milieu,
**caractérisé**
**en ce que** le gaz est introduit à travers une pièce (2) de fumigation suivant l'une des revendications 1 à 4.
